# EUROPEAN PATENT SPECIFICATION

(11) **EP 3 464 612 B1**
(45) Date of publication and mention of the grant of the patent: **30.10.2024**
(21) Application number: 17807182.5
(22) Date of filing: 04.05.2017
(51) Int. Cl.: C12Q 1/06, C12Q 1/6806, G01N 21/62, G01N 33/48, G01N 33/483, C12Q 1/6869, G01N 33/487

(54) **TWO-COLOR NANOPORE SEQUENCING**
ZWEIFARBIGE NANOPORENSEQUENZIERUNG
SÉQUENÇAGE BICOLORE PAR LES NANOPORES

(30) Priority: 31.05.2016 US 201662343283 P; 14.11.2016 US 201662421804 P
(43) Date of publication of application: 10.04.2019
(73) Proprietor: Quantapore, Inc., South San Francisco, CA 94080 (US)
(72) Inventor: GUEGLER, Karl, South San Francisco, CA 94080 (US); SIMONS, Jan F., South San Francisco, CA 94080 (US); MACEVICZ, Stephen C., South San Francisco, CA 94080 (US)
(74) Representative: Boult Wade Tennant LLP
(86) International application number: PCT/US2017/031129
(87) International publication number: WO 2017/209891

(56) References cited:
- WO-A1-2013/014451
- WO-A1-2016/065339
- WO-A1-2016/065339
- WO-A1-2017/100027
- WO-A2-2009/120372
- US-A1- 2009 029 385
- US-A1- 2015 051 099
- US-A1- 2016 011 169
- US-A1- 2016 011 169
- US-A1- 2016 122 818

## Description

### BACKGROUND

Nanopore sequencing has been proposed as an approach to overcome a host of challenges in current DNA sequencing technologies, including reduction of per-run sequencing cost, simplification of sample preparation, reduction of run times, increasing sequence read lengths, providing real-time sample analysis, and the like. However, polymer analysis, such as DNA analysis, with nanopores has its own set of technical difficulties, such as, reliable fabrication of nanostructures for constraining DNA movement, control of DNA translocation rates, unambiguous nucleotide discrimination, detection and processing of signals from large arrays of nanoscale sensors, and so on, e.g. Branton et al, Nature Biotechnology, 26(10): 1146-1153 (2008).

Optical detection of nucleotides has been proposed as a potential solution to some of the technical difficulties in the field of nanopore sequencing, for example, the difficulty of collecting independent signals from large arrays of nanopores. There are numerous challenges, however, to the implementation of such solutions, which include, for example, overcoming background noise typical of optically-based single-molecule analysis and establishing reliable and convenient methods for attaching optical labels to nucleotides.

US 2016/011169 A1 provided for sequencing of nucleic acid templates using nanopores.

Compositions and methods for nucleic acid sequencing provided in WO 2009/120372 A2 include template constructs that comprise double stranded portions in a partially or completely contiguous constructs, to provide for redundant sequence determination through one or both of sequencing sense and antisense strands, and iteratively sequencing the entire construct multiple times.

WO 2013/014451 relates to a method of sequencing a double stranded target polynucleotide. The two strands of the double stranded target polynucleotide are linked by a bridging moiety.

WO 2016/065339 discloses a optically-based nanopore sequencing method.

In view of the above, it would be advantageous to nanopore sequencing technology and its particular applications, such as optically-based nanopore sequencing, if methods were available that would permit optically-based nanopore sequence analysis of target polynucleotides with as few as two different optical labels.

### SUMMARY OF THE INVENTION

The invention provides a method of analyzing double stranded polynucleotides comprising (a) copying a strand of a double stranded polynucleotide so that nucleotide analogs with distinct optical labels are substituted for two or three kinds of nucleotide to form a labeled strand; (b) copying a complement of the strand so that said nucleotide analogs are substituted for the same two or three kinds of nucleotide to form a labeled complement; (c) translocating the labeled strand through a nanopore so that the nucleotides of the labeled strand pass single file through an excitation zone where optical labels are excited to generate optical signals; (e) detecting a time series of optical signals from the optical labels as the labeled strand translocates through the nanopore to produce a strand optical signature; (f) translocating the labeled complement through a nanopore so that the nucleotides of the labeled complement pass single file through an excitation zone where optical labels are excited to generate optical signals; (g) detecting a time series of optical signals from the optical labels as the labeled complement translocates through the nanopore to produce a complement optical signature; (h) determining a sequence of the double stranded polynucleotide from the strand optical signature and the complement optical signature.

The present invention advantageously overcomes the problem of attaching optical labels to each and every nucleotide for optically-based nanopore sequencing. In one aspect, the problem is addressed by labeling a subset of nucleotide types, for example, T's and C's, in both the sense and antisense strands of a target polynucleotide and combining sequence information from optical signatures generated by both strands to obtain a nucleotide sequence for the target polynucleotide. In another aspect, the problem is addressed by labeling a subset of nucleotide types in one or the other strand of a target polynucleotide and comparing optical signatures generated with a reference library of optical signatures derived or measured from known genome sequences. These and other advantages of the present invention are exemplified in a number of implementations and applications, some of which are summarized below and throughout the specification.

### BRIEF DESCRIPTION OF THE DRAWINGS

Figs. 1A-1F illustrate different embodiments of the invention.
Fig. 2 illustrates an optically-based nanopore sequencing system using FRET signal generation and an epi-illumination detection system.
Figs. 3 illustrates an optically-based nanopore sequencing system using FRET signal generation and a TIRF system.
Fig. 4 illustrates the basic components of a confocal epi-illumination system.
Fig. 5 illustrates elements of a TIRF system for excitation of optical labels without FRET signal generation.
Fig. 6 is a flow chart illustrating a step for calling nucleotide sequences based on measurements of optical signals comprising light from multiple optical labels.

### DETAILED DESCRIPTION OF THE INVENTION

Particular nanopore types and numbers, particular labels, FRET pairs, detection schemes, fabrication approaches are shown for purposes of illustration. It should be appreciated that the disclosure is not intended to be limiting in these respects, as other types of optical labels, especially fluorescent labels, and other types of nanopores, arrays of nanopores, and other fabrication technologies may be utilized to implement various aspects of the invention as discussed herein. Guidance for aspects of the invention is found in many available references and treatises well known to those with ordinary skill in the art, including, for example, Cao, Nanostructures & Nanomaterials (Imperial College Press, 2004); Levinson, Principles of Lithography, Second Edition (SPIE Press, 2005); Doering and Nishi, Editors, Handbook of Semiconductor Manufacturing Technology, Second Edition (CRC Press, 2007); Sawyer et al, Electrochemistry for Chemists, 2nd edition (Wiley Interscience, 1995); Bard and Faulkner, Electrochemical Methods: Fundamentals and Applications, 2nd edition (Wiley, 2000); Lakowicz, Principles of Fluorescence Spectroscopy, 3rd edition (Springer, 2006); Hermanson, Bioconjugate Techniques, Second Edition (Academic Press, 2008); and the like.

Described herein but not part of the invention are methods and devices for analyzing polymers comprising linear chains of at least two types of monomers, such as polynucleotides, including DNA, RNA, and the like, using nanopores and optical detection. In some embodiments, as few as two different kinds of nucleotide are labeled with different optical labels that generate distinguishable optical signals for the selected kinds of nucleotide in both sense strands and antisense strands of target polynucleotides. For example, C's and T's of the complementary strands of each target polynucleotide may be replaced by labeled analogs, wherein the labels of the C and T analogs are capable of generating distinct optical signals. Optical signatures are then generated by translocating the labeled strands through nanopores where nucleotides of the strands are constrained to pass sequentially through an optical detection region where their labels are caused to generate optical signals. In some embodiments, information from optical signatures from both sense and antisense strands of a target polynucleotide are combined to determine complete nucleotide sequences of the target polynucleotides. In some embodiments, optical signatures from sense and antisense strands are determined from separate nanopore measurements then combined base on the complementarity of optical signatures. In some such embodiments, such determinations are de novo sequence determinations of the target polynucleotides. In some embodiments, a single nucleotide is labeled so that target polynucleotides generate only single-color optical signatures which may be used to identify a nucleotide sequence in a reference library for a particular organism.

In some embodiments, the selected kinds of nucleotides of target polynucleotides are replaced by labeled nucleotide analogs in an extension reaction using a nucleic acid polymerase. Labeled strands of target polynucleotides are translocated through nanopores that constrain the strands to move single file through an optical detection region where they are excited so that they produce an optical signal indicative of the kind of nucleotide to which the signal-generating label is attached. A collection of optical signals for an individual strand is referred to herein as an optical signature of the strand. In some embodiments, where a strand and its complement (i.e. sense and antisense strands) are linked, for example, via a hairpin adaptor, a single optical signature may include optical signals from optical labels on nucleotides from both the sense strand and the antisense strand. In other embodiments, different strands of a target polynucleotide may separately generate two different optical signatures which may be combined, or used together, for analysis, as mentioned above. Such separately analyzed strands may be associated after generation of optical signatures, for example, by using molecular tags (which may be, for example, oligonucleotide segments which are attached to target polynucleotides in a known position, and which have a length and sequence pattern and diversity to permit ready association). Alternatively, such separately analyzed strands may be associated after generation of optical signatures by their unique sequences of optical signals, i.e. optical signatures, provided that distinct labels are used for a plurality of different kinds of nucleotides on each strand and provided that the target polynucleotide is longer than a predetermined length and the target polynucleotide is drawn from a population below a predetermined size. That is, optical signatures from the two strands of a double stranded target polynucleotide may be correctly associated as being generated from the same original molecule with a predetermined probability that depends on the labeling scheme used (particularly the number of labels used on each strand), the length of the target polynucleotide and the size of the population from which the target polynucleotide is drawn. Such tagging of strands by their unique optical signatures may be referred to herein as an inherent strand tag.

The concept can be illustrated by the following simplified example: let target polynucleotides be members of a population of 10⁸ random-sequence polynucleotides each 100 basepairs in length, and let T's and C's be distinctly labeled on each strand so that signals related to T's, C's and purines were detectable. There would be 3¹⁰⁰ (~5×10⁴⁷) distinct optical signatures that could be measured, so that even with a large sample of 10⁸ sequences the probability is very high that each optical signature measured would be unique and therefore could be uniquely associated with the optical signature of a complementary strand generated from the same target polynucleotide. In some embodiments, two or three kinds of nucleotide have distinct labels (e.g. T's and C's), target polynucleotides are at least 50 basepairs in length, and target polynucleotides are selected from a population of less than 10¹⁰ distinct molecules. In other embodiments, two or three kinds of nucleotide have distinct labels (e.g. T's and C's), target polynucleotides are at least 100 basepairs in length, and target polynucleotides are selected from a population of less than 10¹⁵ distinct molecules of at least 100 basepairs. In other embodiments, two or three kinds of nucleotide have distinct labels (e.g. T's and C's), target polynucleotides are at least 100 basepairs in length, and target polynucleotides are selected from a population of less than 10¹⁰ distinct molecules of at least 100 basepairs. In some embodiments, the number of distinctly labeled nucleotides in each strand, the minimal length of target polynucleotides and the size of the population of target polynucleotides are selected so that optical signatures generated from complementary strands of the same target polynucleotide may be correctly associated, or identified, with a probability of at least 95 percent, or with a probability of at least 99 percent, or with a probability of at least 99.9 percent. In some embodiments, the size of the population of target polynucleotides may be a predetermined size, or it may be simply a known size of population, or it may be an estimated size of a population. In each of these cases, the other parameters (number of labels, minimal signature length, and the like) are selected to obtain the desired probabilities of correct association or pairing of optical signatures from complementary strands.

As used herein, in some embodiments (e.g. using only two labels), the terms "associating" or "pairing" in reference to optical signatures of candidate complementary strands means matching patterns of signals from one strand to patterns of intervals between signals (possibly in reverse temporal order depending on the embodiment) from another strand. In embodiments in which three labels are used (e.g. C with label 1, T with label 2, and A or G with label 3), instead of intervals between signals (e.g. C or T signals) there would be sequences of "A or G" signals from label 3.

In further embodiments, if only two kinds of nucleotide are labeled and detectable, e.g. T's and C's, the number of possible different optical signatures from such labeled strands is still large enough to be used as inherent strand tags. For example, in a population of random-sequence polynucleotides with lengths of 100 basepairs, labeled T's and C's would generate about 2⁵⁰, or about 10¹⁵, distinct optical signatures.

One of ordinary skill in the art would understand that the determination of the number of distinct inherent strand tags in any given circumstance depends in part on the magnitude of noise from several sources, for example, intra-strand variation in translocation speeds of nucleotides, variations in the volume of detection regions from which optical signals are collected, local physical and chemical influences on optical signal magnitude or quality, and the like. The effects on optical signatures of noise from such sources may be treated by conventional data analysis techniques.

As noted below, optical signatures may comprise mixed optical signals in that the signal detected in each detection interval may comprise contributions from multiple optical labels emitting within a resolution limited area or volume; that is, they may (for example) be mixed FRET signals, as described by Huber et al, U.S. patent publication US20160076091.

Described herein but not part of the invention is a method implemented with the following steps: (a) copying a strand of a double stranded polynucleotide so that nucleotide analogs with distinct optical labels are substituted for at least two kinds of nucleotide to form a labeled strand; (b) copying a complement of the strand so that said nucleotide analogs are substituted for the same at least two kinds of nucleotide to form a labeled complement; (c) translocating the labeled strand through a nanopore so that the nucleotides of the labeled strand pass single file through an excitation zone where optical labels are excited to generate optical signals; (d) detecting a time series of optical signals from the optical labels as the labeled strand translocates through the nanopore to produce a strand optical signature; (e) translocating the labeled complement through a nanopore so that the nucleotides of the labeled complement pass single file through an excitation zone where optical labels are excited to generate optical signals; (f) detecting a time series of optical signals from the optical labels as the labeled complement translocates through the nanopore to produce a complement optical signature; (g) determining a sequence of the double stranded polynucleotide from the strand optical signature and the complement optical signature. In some embodiments, two kinds of nucleotide are labeled, which may be C's and T's, C's and G's, C's and A's, T's and G's, T's and A's, or G's and A's. In some embodiments, pyrimidine nucleotides are labeled. In other embodiments, purine nucleotides are labeled. In some embodiments, selected kinds of nucleotides of a strand are labeled by incorporating labeled analog dNTPs of the selected kind of nucleotides in a primer extension reaction using a nucleic acid polymerase. In other embodiments, selected kinds of nucleotides of a strand are labeled by incorporating analog dNTPs of the selected kinds of nucleotides in an extension reaction, wherein the analog dNTPs are derivatized with orthogonally reactive functionalities that allow attachment of different labels to different kinds of nucleotides in a subsequent reaction. This latter labeling approach is disclosed in Jett et al, U.S. patent 5,405,747.

In some embodiments, three kinds of nucleotide are labeled, which may include labeling C's with a first optical label, T's with a second optical label, and G's and A's with a third optical label. In other embodiments, the following groups of nucleotides may be labeled as indicated: C's and G's with a first optical label and second optical label, respectively, and T's and A's with a third optical label; C's and A's with a first optical label and second optical label, respectively, and T's and G's with a third optical label; T's and G's with a first optical label and second optical label, respectively, and C's and A's with a third optical label; A's and G's with a first optical label and second optical label, respectively, and T's and C's with a third optical label.

In some embodiments, optical labels are fluorescent acceptor molecules that generate a fluorescent resonance energy transfer (FRET) signal after energy transfer from a donor associated with a nanopore. In some embodiments, as described further below, donors may be optically active nanoparticles, such as, quantum dots, nanodiamonds, or the like. Selection of particular combinations of acceptor molecules and donors are design choices for one of ordinary skill in the art. In some embodiments, some of which are described more fully below, a single quantum dot is attached to a nanopore and is excited to fluoresce using an excitation beam whose wavelength is sufficiently separated, usually lower (i.e. bluer), so that it does not contribute to FRET signals generated by acceptors. Likewise, a quantum dot is selected whose emission wavelength overlaps the absorption bands of both acceptor molecules to facilitate FRET interactions. In some embodiments, two donors may be used for each excitation zone of a nanopore, wherein the emission wavelength of each is selected to optimally overlap the absorption band of a different one of the acceptor molecules.

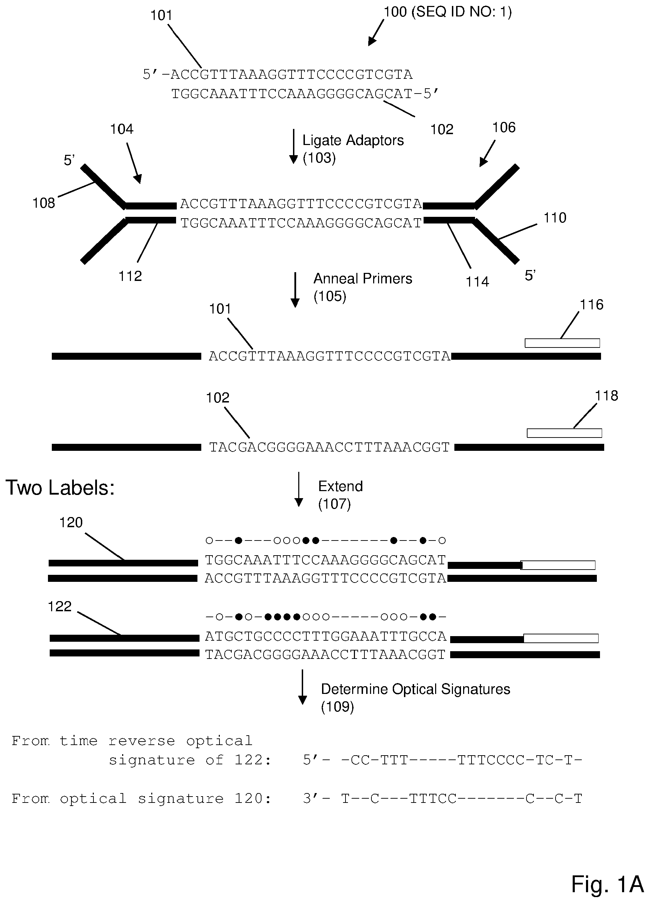

Figs. 1A-1E illustrate several embodiments of the above methods. In Fig. 1A, double stranded target polynucleotide (100) (SEQ ID NO: 1) consists of sense strand (101) and complementary antisense strand (102), to which is ligated (103) "Y" adaptors (104) and (106) using conventional methods, e.g. Weissman et al, U.S. patent 6,287,825; Schmitt et al, U.S. patent publication US2015/004468. Arms (108) and (110) of adaptors (104 and 106, respectively) include primer binding sites to which primers (116) and (118) are annealed (105). Double stranded portions (112) and (114) may include tag sequences, e.g. one or both may include randomers of predetermined length and composition, which may be used for later re-association of the strands, for example, to obtain sequence information from the respective optical signatures of the strands. After annealing primers (116) and (118), they may be extended (107) by a nucleic acid polymerase in the presence of (for example, as illustrated) labeled dUTP analogs (labels shown as open circles in the incorporated nucleotides) and labeled dCTP analogs (labels shown as filled circles in the incorporated nucleotides) and natural unlabeled dGTPs and dATPs (with neither unlabeled dTTP nor unlabeled dCTP being present so that the analogs are fully substituted in the extended strands). The absence of labels on G's and A's are illustrated as dashes above the incorporated nucleotides. In an ideal detection system without noise, the sequence of open circles, filled circles and dashes would be good representations of optical signatures generated by the indicated sense and antisense strands as they pass through an excitation zone of a nanopore. Although in this embodiment strands translocate nanopores 5'-first, the translocation orientation (that is, 5'-first or 3'-first) may be selected in other embodiments to produce optical signature that are not necessarily generated by reverse complements. Or, alternatively, translocation orientation may be selected so that complementary optical signatures are based on both sequences translocating 3'-first.

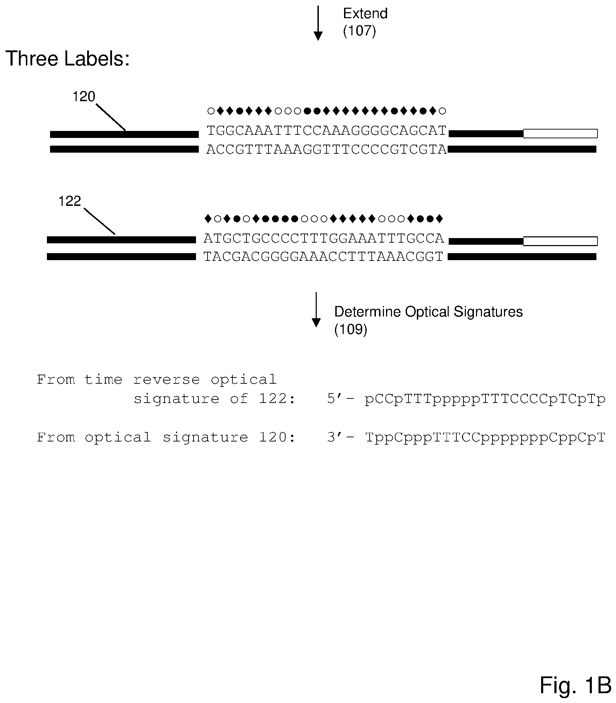

In Fig. 1B, extension products (120) and (122) are illustrated for an alternative embodiment employing three labels. Incorporated labeled dUTP analogs are shown as open circles and incorporated labeled dCTP analogs are shown as filled circles, as above. Incorporated labeled dATP and dGTP analogs are shown as filled diamonds (and below in the nucleotide sequence representation are each shown as "p" for "purine"). A three-label embodiment would have the advantage of providing a basis for quantifying the gaps (or number of nucleotide) between signals for "C's" or "T's." For example, a cumulative signal from a series of contiguous purines would provide a measure for the number of purines, which could be matched with a contiguous segment of C's and T's on a candidate complementary sequence.

In variants of both the embodiments of Fig. 1A and 1B, deduction of nucleotide sequences may be facilitated by first comparing optical signatures of candidate complementary sequences to reference sequences.

Guidance in selecting the kinds of nucleotide to label, kinds of labels and linkers for attaching them to bases, and nucleic acid polymerases for extension reactions in the presence of dNTP analogs can be found in the following references: Goodman et al, U.S. patent 5,945,312; Jett et al, U.S. patent 5,405,747; Muehlegger et al, U.S. patent publication US2004/0214221; Giller et al, Nucleic Acids Research, 31(10): 2630-2635 (2003); Tasara et al, Nucleic Acids Research, 31(10): 2636-2646 (2003); Augustin et al, J. Biotechnology, 86: 289-301 (2001); Brakmann, Current Pharmacuetical Biotechnology, 5(1): 119-126 (2004); and the like. Exemplary nucleic acid polymerases for use with the invention include, but are not limited to, Vent exo⁻, Taq, E. coli Pol I, Tgo exo⁻, Klenow fragment exo⁻, Deep Vent exo⁻, and the like. In some embodiments, exemplary nucleic acid polymerases include, but are not limited to, Vent exo⁻ and Klenow fragment exo⁻. Exemplary fluorescent labels for dNTP analogs include, but are not limited to, Alexa 488, AMCA, Atto 655, Cy3, Cy5, Evoblue 30, fluorescein, Gnothis blue 1, Gnothis blue 2, Gnothis blue 3, Dy630, Dy635, MR121, rhodamine, Rhodamine Green, Oregon Green, TAMRA, and the like. Exemplary fluorescent labels for dUTP analogs include, but are not limited to, Alexa 488, AMCA, Atto 655, Cy3, Cy5, Dy630, Dy665, Evoblue 30, Evoblue 90, fluorescein, Gnothis blue 1, Gnothis blue 2, Gnothis blue 3, MR121, Oregon Green, rhodamine, Rhodamine Green, TAMRA, and the like. Exemplary fluorescent labels for dCTP analogs include, but are not limited to, Atto 655, Cy5, Evoblue 30, Gnothis blue 3, rhodamine, Rhodamine Green, TAMRA, and the like. Exemplary fluorescent labels for dATP analogs include, but are not limited to, Atto 655, Cy5, Evoblue 30, Gnothis blue 3, Rhodamine Green, and the like. Exemplary fluorescent labels for dGTP analogs include, but are not limited to, Evoblue 30, Gnothis blue 3, Rhodamine Green, and the like. Exemplary pairs of fluorescent labels for dUTP analogs and dCTP analogs include, but are not limited to, (TAMRA, Rhodamine Green), (Atto 655, Evoblue 30), (Evoblue 30, Atto 655), (Evoblue 30, Gnothis blue 3), (Evoblue 30, Rhodamine Green), (Gnothis blue 1, Rhodamine Green), (Gnothis blue 2, Atto 655), Gnothis blue 3, Cy5), and the like.

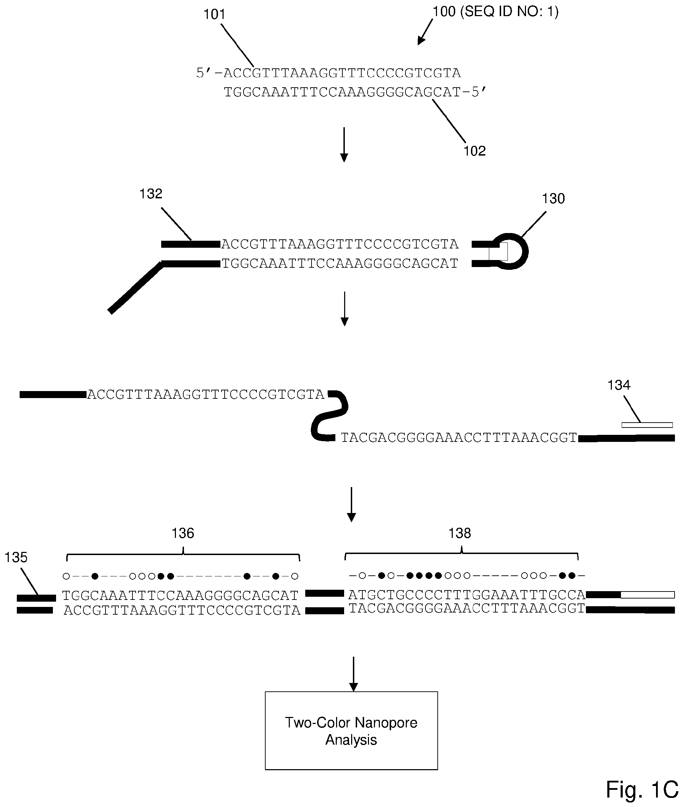

Fig. 1C illustrates an embodiment in which two labels are used and sense and antisense strands are linked by means of hairpin adaptor (130), for example, as taught in U.S. patent publications US 2015/0152492 and US 2012/0058468. Tailed adaptor (132) and hairpin adaptor (130) are ligated to target polynucleotide (100) (SEQ ID NO: 1). After denaturation and annealing of primer (134), an extension reaction produces extension product (135) which includes segment (136) (which is the labeled complement of strand (101)) and segment (138(which is the labeled reverse complement of strand (101)). After translocation of extension product (135) through a nanopore and generation of an optical signature the sequence of target polynucleotide (100) can be determined. Optionally, the sequence of hairpin (130) may be selected so that a predetermined pattern of labels is incorporated during the extension reaction, which may be used to assist in the analysis of the optical signature, e.g. by indicating where segment (136) ends and where segment (138) begins, or the like.

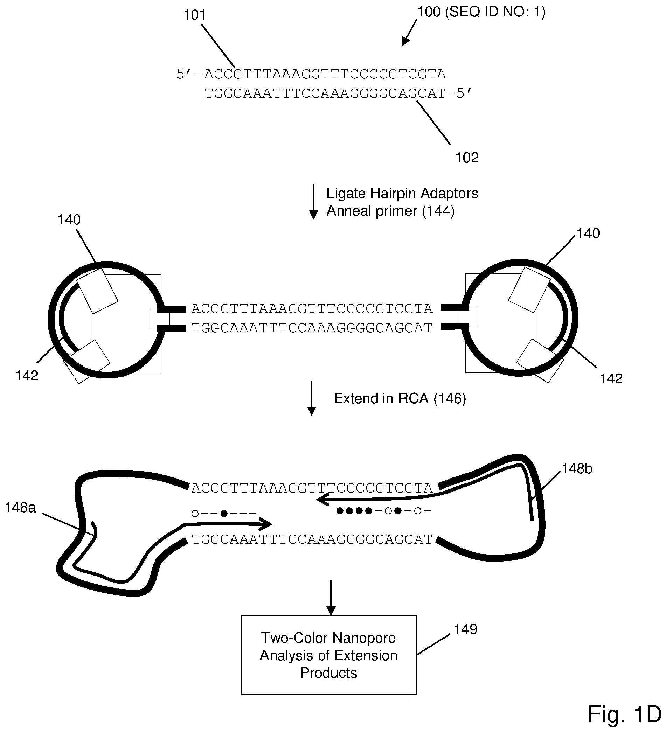

Fig. 1D illustrates another embodiment where two identical hairpin adaptors (140) are ligated to the ends of target polynucleotide (100) (SEQ ID NO: 1). In this embodiment, hairpin adaptors (140) include primer binding sites to which primers (142) may anneal (144) and be extended in a rolling circle amplification (RCA) to produce labeled extension products (148a and 148b). In some embodiments, RCA (146) is performed so that at least one complete circuit is made of sense strand (101) and antisense strand (102). Extension products (148a and 148b) are then translocated through nanopores so that labeled nucleotides pass single file through an excitation zone for generating optical signatures.

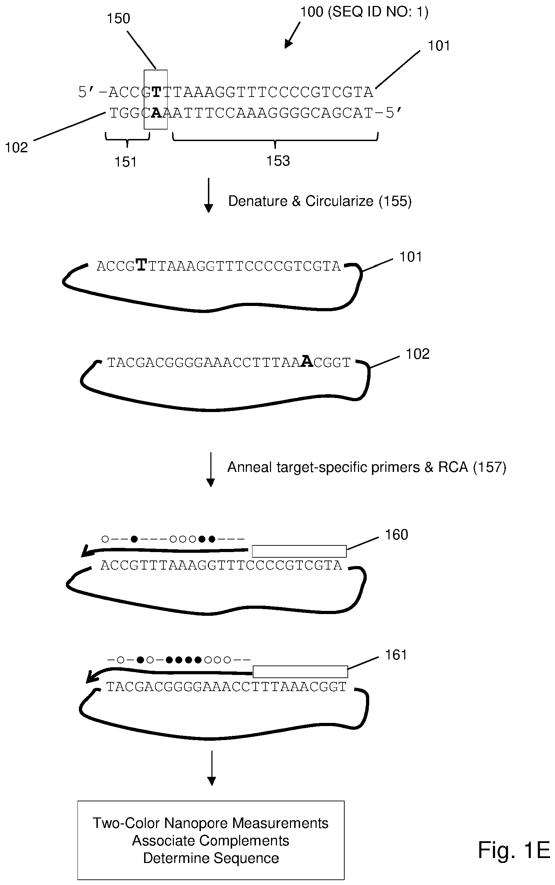

Fig. 1E illustrates a measurement in which target polynucleotides (100) (SEQ ID NO: 1) contain a genetic locus (150) containing or consisting of, for example, a single nucleotide polymorphism (SNP), but which otherwise comprises a known sequence. The object of the measurement is to determine the identity of the SNP following a method similar to that disclosed by Nelson et al, U.S. patent 8921072; U.S. patent publications US2015/0031035 and US20015/0031086; Lin et al, International patent publication WO2015/089333; Lou et al, Proc. Natl. Acad. Sci., 110(49): 19872-19877 (2013). That is, in this embodiment, rolling circle replication of the locus of interest (and its complement) allows one to determine the identity of locus (150) with greater reliability and with a reduced error rate. Target polynucleotide (100) is denatured and circularized (155) as taught in the above cited references, after which target specific primers (160) and (161) are annealed to single stranded circles containing sequences (101) and (102). Target specific primers (160) and (161) are extended in the presence of selected nucleotide analogs, e.g., labeled dUTPs and labeled dCTPs, after which optical signatures are generated. Sense (101) and antisense (102) strands may be associated by means of an inherent tag of target polynucleotide (100) which consists of the unique relative position of genetic locus (150) in polynucleotide (100). In the example of Fig. 1E, the sequences of segment (151) and segment (153) and the length of polynucleotide (100) allows identification of the sense strand and antisense strands. Alternatively, adaptors with one or more molecular tags may be ligated to target polynucleotide (100) prior to denaturation and circularization.

Described herein but not part of the invention are methods for measuring optical signatures that allow identification of labeled target polynucleotides without providing a complete nucleotide sequence. In particular, such methods may be used for expression profiling. Such methods may be carried out by the following steps: (a) obtaining a sample of RNA; (b) reverse transcribing strands of mRNA so that nucleotide analogs with distinct optical labels are substituted for at least two kinds of nucleotide to form a labeled first strand; (c) translocating the labeled first strand through a nanopore so that the nucleotides of the labeled strand pass single file through an excitation zone where optical labels are excited to generate optical signals; (d) detecting a time series of optical signals from the optical labels as the labeled first strand translocates through the nanopore to produce a first strand optical signature; (e) determining a sequence of the RNA strands from the first strand optical signature by comparing the first strand optical signature to reference optical signatures.

As used herein, "reference optical signatures" mean a collection or library or database of optical signatures produced from known polynucleotides which had standard labels on predetermined kinds of nucleotides and which were measured under standard conditions, so that an experimental optical signature obtained under the same conditions (or conditions that permit rational conversion of experimental signal values to database signal values) can be related to and/or identified with a reference optical signature. In some instances, optical signatures may be first processed to determine nucleotide sequences (that is, "base called" or converted into nucleotide sequences) of only the labeled nucleotides (e.g., ignoring the identities of the unlabeled nucleotides). Such "signature" nucleotide sequences would then be compared to a library of reference signature sequences for identification. Such libraries of reference signature sequences may be for specific organisms or group of organisms, e.g. human (genome, transcriptome, etc.), microorganisms, bacteria, and so on.

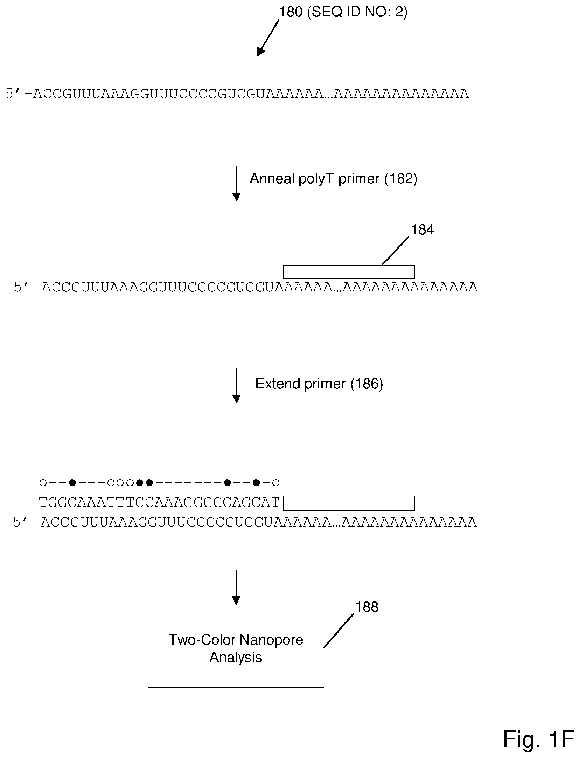

Fig. 1F illustrates the above method. PolyT primer (184) is annealed (182) to polyA tail of mRNA (180) (SEQ ID NO: 2), after which it is extended by a reverse transcriptase in the presence of labeled dUTP and labeled dCTP to produce extension product (185). Extension product (185) is then isolated and analyzed by translocation through a nanopore to produce an optical signature which is compared to a database of reference signatures for identification (188). It is understood by one of ordinary skill in the art that the length of RNA (180) or other target polynucleotide would have to have a minimal length to generate an optical signature that had a high probability of being uniquely identified by comparison to database signatures. In some instances, target polynucleotides or RNAs have lengths of at least 100 nucleotides, or at least 200 nucleotides, or at least 300 nucleotides. In other instances, such target polynucleotides or RNAs have lengths of at least 500 nucleotides. In some instances, RNA analytes are used to generate DNAs having at least two kinds of nucleotide labeled with distinct fluorescent labels. In further instances, such RNA analytes have a minimal length for identification which is selected so that a gene encoding an RNA analyte is identified with a probability of at least ninety-five percent, or with a probability of at least ninety-nine percent. In some instances, the at least two kinds of nucleotide are C's and T's. In some instances, RNA analytes are obtained from human samples, e.g. blood samples, or other tissue samples.

### Optical Signal Detection

In some embodiments, a series of optical signals may be measured from a resolution limited area wherein each optical measurement comprises a plurality of component signals from different adjacent monomers (whose order in the polymer cannot be determined from a single measurement because, for example, the component signals are generated from within a diffraction limited area). Under these circumstances, optically-based nanopore analysis of polymers (i) generates a time series of optical measurements that comprise overlapping contributions from sequences of more than one labeled monomer, thereby making it difficult, if not impossible, to determine an ordering of the monomers from a single measurement, and (ii) by selecting optical labels for monomers which generate distinguishable signals, the optical measurements can be separated into contributions from different labels on different kinds of monomers, which allows overlapping measurements to be converted into sequence information.

Described herein but not part of the invention is a method implemented by the following steps: (a) translocating a polymer through a nanopore, wherein different kinds of monomers of the polymer are labeled with different optical labels that generate distinguishable optical signals and wherein the nanopore constrains the monomers to move single file through an excitation zone that encompasses a plurality of monomers; (b) detecting a time series of optical signals from the monomers as the polymer passes through the excitation zone; (c) separating optical signals from different kinds of monomers; and (d) determining a sequence of monomers from time series of separated optical signals from the polymer.

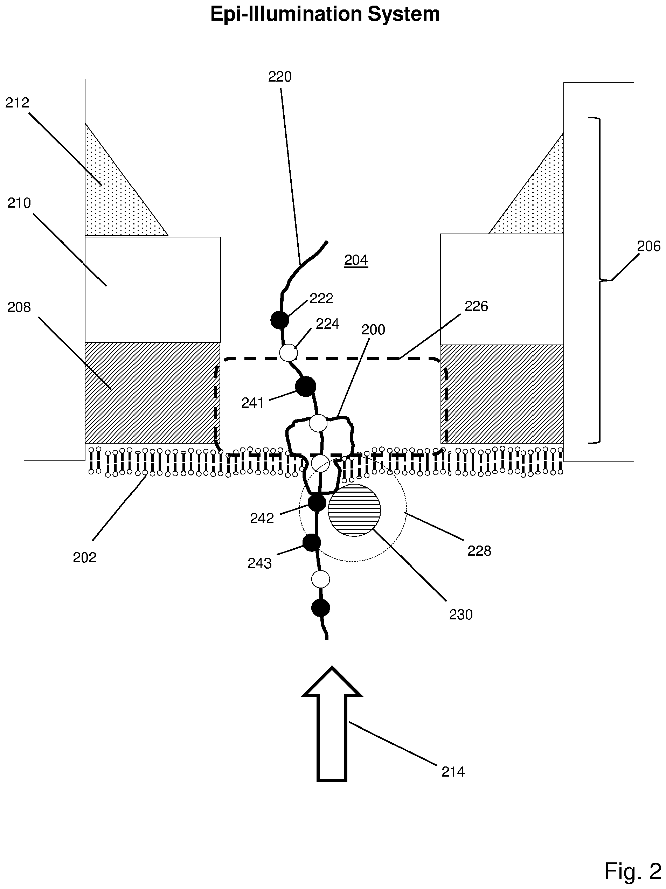

In some embodiments, optical signals may be FRET signals or they may be fluorescent emissions from directly excited fluorescent labels attached to monomers. Fig. 2 illustrates components of one embodiment in which a protein nanopore (200) is disposed in a lipid bilayer (202) disposed (in turn) across aperture (204) of solid state membrane (206), which comprises opaque layer (208) (such as a metal layer), silicon nitride layer (210) and silicon support layer (212). Opaque layer (208) prevents or reduces transmission of excitation beam (214) through solid state membrane (206) where it could excite undesired background fluorescence. As polymer (220) with differently labeled monomers (illustrated as black (222) and white (224)) pass through nanopore (200), at each measurement interval a plurality of monomers (such as, 241, 242 and 243) are present in excitation zones (226) and (228) within the same resolution limited area. In the illustrated embodiment, optical measurements are made with an epi-illumination system and it is assumed that nanopore (200) has been selected so that optical signals from monomers interior to nanopore (200) are suppressed and do not contribute to measured optical signals. Excitation zone (228) is a FRET zone adjacent to FRET donor (230); that is, excitation zone (228) defines a distance from FRET donor (230) within which FRET can occur between FRET donor (230) and an optical label attached to a monomer, which may also be referred to as an acceptor label, or FRET acceptor label. Excitation zone (226) is a non-propagating protrusion of a component of excitation beam (214) into aperture (204) which occurs whenever the dimensions of aperture (204) are selected to be sufficiently below the wavelength of excitation beam (214). As illustrated, in this embodiment, a plurality of monomers (241, 242 and 243) would contribute to an optical signal measured at the instant, or interval, during which monomers (241, 242 and 243) are in the excitation zones (226) and (228).

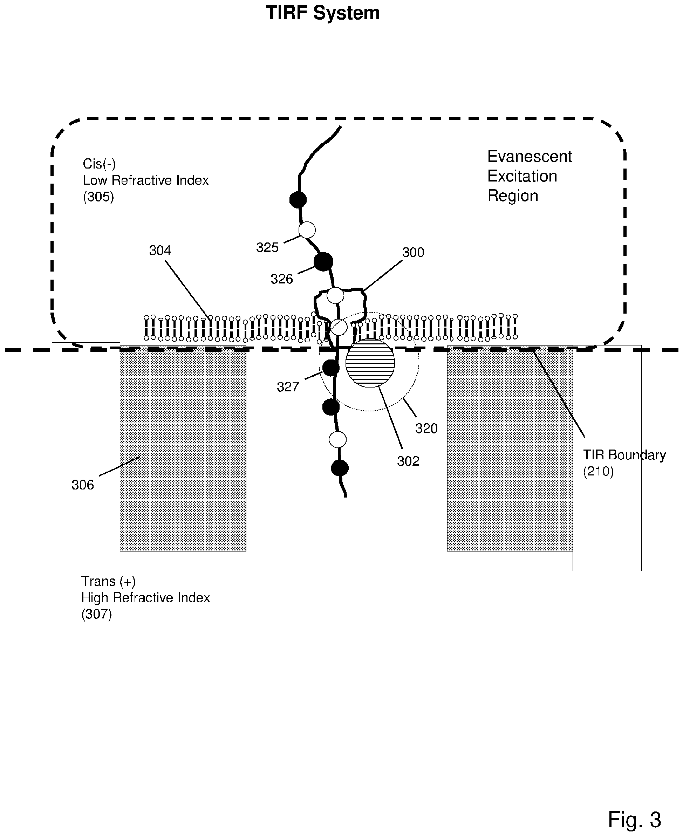

Fig. 3 illustrates an embodiment in which optical measurements are made with total internal reflection fluorescence (TIRF) excitation in a system such as described in Soni et al, Review of Scientific Instruments, 81: 014301 (2010); and in U.S. patent publication 2012/0135410. In this embodiment, protein nanopore (300) with attached FRET donor (302) is inserted into lipid bilayer (304) disposed on solid state membrane (306) with aperture (308). Total internal reflection (TIR) is made possible by selecting electrolytes on cis (305) and trans (307) sides of solid state membrane (306) with different indices of refraction. As a result, TIR boundary (310) is created at or near the plane that solid state membrane (306) is disposed in, so that an evanescent field is created on the cis (305) side of solid state membrane (306). The evanescent field may excite optical labels prior to their entry into nanopore (300). FRET donor (302) is excited directly by light reflected at the TIR boundary (310), so that FRET can take place between FRET donor (302) and labels on monomers (319) within FRET zone (320). As in the embodiment of Fig. 2, nanopore (300) may be selected so that fluorescent emissions by labels are suppressed when labeled monomers are in the bore of nanopore (300). A plurality of monomers, such as 325, 326 and 327, contribute to an optical measurement recorded at the indicated configuration in the figure.

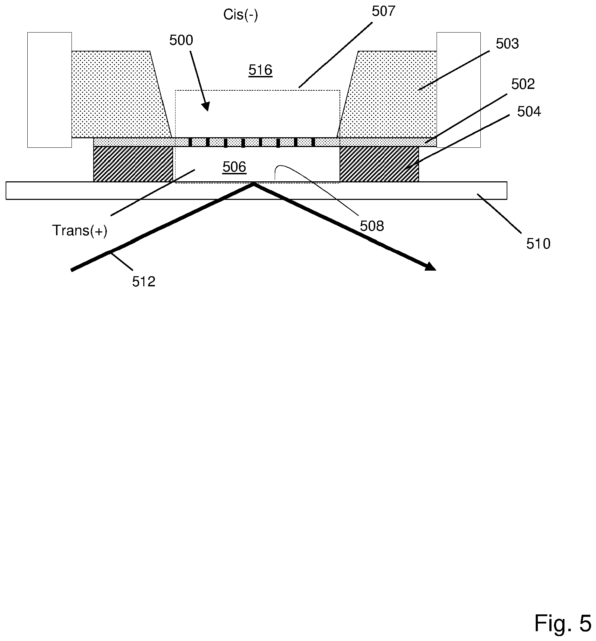

In some embodiments, labels on monomers may be excited by an evanescence field alone using an apparatus similar to that shown in Fig. 5. In this apparatus, a very narrow second chamber on the trans side of a nanopore or nanopore array permits an evanescent field to extend from a surface of an underlying glass slide to establish excitation zones both at entrances and exits of the nanopores, so that each optical measurement associated with a nanopore contains contributions from a plurality of labeled monomers. Array of apertures (500) (which may include protein nanopores inserted in a lipid bilayer), may be formed in silicon nitride layer (502), which may have a thickness in the range of from 20-100 nm. Silicon nitride layer (502) may be formed on a silicon support layer (503). Second chamber (506) may be formed by silicon nitride layer (502), silicon dioxide layer (504) which determines the height of second chamber (506), and surface (508) of glass slide (510). Silicon dioxide layer (504) may have a thickness in the range of from 50-100 nm. A desired evanescent field (507) extending from surface (508) across silicon nitride layer (502) may be established by directing light beam (512) at an appropriate angle relative to glass slide (510) so that TIR occurs. For driving labeled polynucleotide analytes through array (500), cis(-) conditions may be established in first chamber (516) and trans(+) conditions may be established in second chamber (506) with electrodes operationally connected to first and second chambers (506 and 521).

### Sequence Determination with Mixed FRET Signals

In accordance with the invention, when a labeled polymer translocates through a nanopore and its associated excitation zones, a time-ordered set of optical measurements are recorded. Optical measurements at adj acent time points are overlapping in the sense that each optical measurement contains contributions from labels of adjacent monomers. Thus, for example, if three monomers generate signals at each time point (for example, B, C and D of polymer ... -A-(B-C-D)-... moving through an excitation zone from left to right), and if one monomer exits the excitation zone and another monomer enters the excitation zone (indicated by parentheses) between successive measurements (for example, A enters and D exits: -(A-B-C)-D...), then two successive optical measurements will contain contributions from the same monomers (in this example, both measurements include contributions from B and C. The above example is based on a very simplified model of polymer translocation through nanopores; however, the concept of successive overlapping optical measurements is applicable to more complex descriptions of polymer translocation.

Since emissions from a plurality of different labeled monomers at a nanopore originate from the same resolution limited area, relative position information (in particular, sequence information) about the monomers cannot be determined from a single optical measurement. However, because of the overlap and the use of labels that generate monomer-specific signals, in some embodiments, sequence information may be determined from the time-ordered set of optical signal measurements when it is separated into a plurality of time-ordered sets of monomer-specific signals. Algorithms similar to those used in sequencing-by-hybridization (SBH) to reconstruct target polynucleotide sequences from hybridization data may be used to reconstruct target polynucleotides here, e.g. U.S. patent 5,002,867; Timp et al, Biophys. J., 102: L37-L39 (2012). The constraints of (i) time-ordered overlapping signals and (ii) their separation into monomer-specific components significantly simplify the determination step in the case of optical detection.

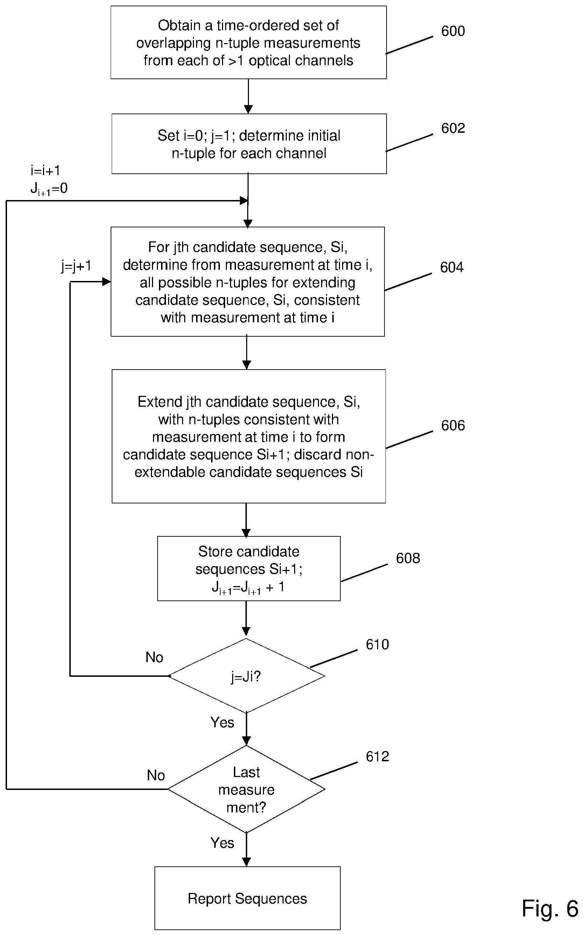

Fig. 6 illustrates one embodiment of a step for determining monomer sequence information from a time-ordered set of overlapping optical signals based on a simple model of nanopore translocation. The simple model assumes that optical measurements at each time step (except at the entry and exit of a polymer from a nanopore) each contain signal contributions from the same number of monomers (referred to in Fig. 6 as an "n-tuple" to indicate that a measurement would contain contributions from n monomers). It is understood that more complex models may allow for differing numbers of contributing monomers in each measurement, for local variations in translocation speed, deviations in linear movement of monomers, and other like phenomena. That is, in some embodiments, optical measurements at different times may have contributions from different numbers of nucleotides. In some embodiments, the differing number of nucleotides are ordered along a segment of the target polynucleotide. The step of determining illustrated by Fig. 6 assumes that a labeled polymer has passed through a nanopore and that a time ordered set of optical measurements has been made, including separation of optical signals into monomer-specific signals (600). The entry and exit of a polymer are treated differently since there are necessarily different numbers of monomers in the excitation zone(s) upon entry and exit. In this embodiment, it is assumed that initial and final optical measurements under these conditions permits the initial and final monomers to be determined directly from their monomer-specific signal. In other embodiments, preparation of labeled polymers for analysis may include insertion of a plurality of predetermined labeled nucleotides at one or both ends of such labeled polymers for the purpose of generating a known sequence of optical signals to aid in a sequence determination step. Such predetermined labeled nucleotides would be similar to key sequences in Ion Torrent or 454 sequencing, e.g. U.S. patent 7,575,865.

Returning to Fig. 6, at the beginning of a determining step, time index, i, is set to zero; the index, j, for candidate sequences at the current time, i, is set to 1 (602); and the initial n-tuple of the set of monomer-specific time-ordered optical signals is examined (604). Such examination comprises first determining from the measurement at time i all possible n-tuples of monomers that are consistent with the measurement, then determining from those n-tuples which ones that properly overlap candidate sequence Si. New candidate sequences Si+1 are formed (and a sequence Si is extended) by each properly overlapping n-tuple for the set consistent with the measurement (606). New extended candidate sequences, Si+1, are stored and the index giving the number of candidate sequences at time i+1, Ji+1, is updated (608). This step is repeated until every candidate sequence, Si, has been examined (610), and a similar examination is carried out at each time, i, until each optical measurement in the time-ordered set has been examined.

### Nanopores and Nanopore Arrays

Nanopores used with the invention may be solid-state nanopores, protein nanopores, or hybrid nanopores comprising protein nanopores or organic nanotubes such as carbon or graphene nanotubes, configured in a solid-state membrane, or like framework. Important features of nanopores include constraining polymer analytes, such as polynucleotides, so that their monomers pass through a signal generation region (or excitation zone, or the like) in sequence, That is, so that monomers, such as nucleotides, pass through a detection zone (or excitation region or like region) in single file. In some embodiments, additional features of nanopores include passing single stranded nucleic acids while not passing double stranded nucleic acids, or equivalently bulky molecules. In other embodiments, nanopores, especially protein nanopores, may be selected so that their bores are sized so that labels of monomers are sterically constrained so that FRET signals, or even fluorescent signals, are suppressed.

In some embodiments, nanopores used in connection with the methods of the invention are provided in the form of arrays, such as an array of clusters of nanopores, which may be disposed regularly on a planar surface. In some embodiments, clusters are each in a separate resolution limited area so that optical signals from nanopores of different clusters are distinguishable by the optical detection system employed, but optical signals from nanopores within the same cluster cannot necessarily be assigned to a specific nanopore within such cluster by the optical detection system employed.

Solid state nanopores may be fabricated in a variety of materials including but not limited to, silicon nitride (Si₃N₄), silicon dioxide (SiO₂), and the like. The fabrication and operation of nanopores for analytical applications, such as DNA sequencing, are disclosed in the following exemplary references: Ling, U.S. patent 7,678,562; Hu et al, U.S. patent 7,397,232; Golovchenko et al, U.S. patent 6,464,842; Chu et al, U.S. patent 5,798,042; Sauer et al, U.S. patent 7,001,792; Su et al, U.S. patent 7,744,816; Church et al, U.S. patent 5,795,782; Bayley et al, U.S. patent 6,426,231; Akeson et al, U.S. patent 7,189,503; Bayley et al, U.S. patent 6,916,665; Akeson et al, U.S. patent 6,267,872; Meller et al, U.S. patent publication 2009/0029477; Howorka et al, International patent publication WO2009/007743; Brown et al, International patent publication WO2011/067559; Meller et al, International patent publication WO2009/020682; Polonsky et al, International patent publication WO2008/092760; Van der Zaag et al, International patent publication WO2010/007537; Yan et al, Nano Letters, 5(6): 1129-1134 (2005); Iqbal et al, Nature Nanotechnology, 2: 243-248 (2007); Wanunu et al, Nano Letters, 7(6): 1580-1585 (2007); Dekker, Nature Nanotechnology, 2: 209-215 (2007); Storm et al, Nature Materials, 2: 537-540 (2003); Wu et al, Electrophoresis, 29(13): 2754-2759 (2008); Nakane et al, Electrophoresis, 23: 2592-2601 (2002); Zhe et al, J. Micromech. Microeng., 17: 304-313 (2007); Henriquez et al, The Analyst, 129: 478-482 (2004); Jagtiani et al, J. Micromech. Microeng., 16: 1530-1539 (2006); Nakane et al, J. Phys. Condens. Matter, 15 R1365-R1393 (2003); DeBlois et al, Rev. Sci. Instruments, 41(7): 909-916 (1970); Clarke et al, Nature Nanotechnology, 4(4): 265-270 (2009); Bayley et al, U.S. patent publication 2003/0215881; and the like.

In some embodiments, the invention uses nanopore arrays with one or more light-blocking layers, that is, one or more opaque layers. Typically nanopore arrays are fabricated in thin sheets of material, such as, silicon, silicon nitride, silicon oxide, aluminum oxide, or the like, which readily transmit light, particularly at the thicknesses used, e.g. less than 50-100 nm. For electrical detection of analytes this is not a problem. However, in optically-based detection of labeled molecules translocating nanopores, light transmitted through an array invariably excites materials outside of intended reaction sites, thus generates optical noise, for example, from nonspecific background fluorescence, fluorescence from labels of molecules that have not yet entered a nanopore, or the like. In one aspect, this problem is addressed by providing nanopore arrays with one or more light-blocking layers that reflect and/or absorb light from an excitation beam, thereby reducing background noise for optical signals generated at intended reaction sites associated with nanopores of an array. In some embodiments, this permits optical labels in intended reaction sites to be excited by direct illumination. In some embodiments, an opaque layer may be a metal layer. Such metal layer may comprise Sn, Al, V, Ti, Ni, Mo, Ta, W, Au, Ag or Cu. In some embodiments such metal layer may comprise Al, Au, Ag or Cu. In still other embodiments, such metal layer may comprise aluminum or gold, or may comprise solely aluminum. The thickness of an opaque layer may vary widely and depends on the physical and chemical properties of material composing the layer. In some embodiments, the thickness of an opaque layer may be at least 5 nm, or at least 10 nm, or at least 40 nm. In other embodiments, the thickness of an opaque layer may be in the range of from 5-100 nm; in other embodiments, the thickness of an opaque layer may be in the range of from 10-80 nm. An opaque layer need not block (i.e. reflect or absorb) 100 percent of the light from an excitation beam. In some embodiments, an opaque layer may block at least 10 percent of incident light from an excitation beam; in other embodiments, an opaque layer may block at least 50 percent of incident light from an excitation beam.

Opaque layers or coatings may be fabricated on solid state membranes by a variety of techniques known in the art. Material deposition techniques may be used including chemical vapor deposition, electrodeposition, epitaxy, thermal oxidation, physical vapor deposition, including evaporation and sputtering, casting, and the like. In some embodiments, atomic layer deposition may be used, e.g. U.S. patent 6,464,842; Wei et al, Small, 6(13): 1406-1414 (2010).

In some embodiments, a 1-100 nm channel or aperture may be formed through a solid substrate, usually a planar substrate, such as a membrane, through which an analyte, such as single stranded DNA, is induced to translocate. In other embodiments, a 2-50 nm channel or aperture is formed through a substrate; and in still other embodiments, a 2-30 nm, or a 2-20 nm, or a 3-30 nm, or a 3-20 nm, or a 3-10 nm channel or aperture if formed through a substrate. The solid-state approach of generating nanopores offers robustness and durability as well as the ability to tune the size and shape of the nanopore, the ability to fabricate high-density arrays of nanopores on a wafer scale, superior mechanical, chemical and thermal characteristics compared with lipid-based systems, and the possibility of integrating with electronic or optical readout techniques. Biological nanopores on the other hand provide reproducible narrow bores, or lumens, especially in the 1-10 nanometer range, as well as techniques for tailoring the physical and/or chemical properties of the nanopore and for directly or indirectly attaching groups or elements, such as fluorescent labels, which may be FRET donors or acceptors, by conventional protein engineering methods. Protein nanopores typically rely on delicate lipid bilayers for mechanical support, and the fabrication of solid-state nanopores with precise dimensions remains challenging. In some embodiments, solid-state nanopores may be combined with a biological nanopore to form a so-called "hybrid" nanopore that overcomes some of these shortcomings, thereby providing the precision of a biological pore protein with the stability of a solid state nanopore. For optical read out techniques a hybrid nanopore provides a precise location of the nanopore which simplifies the data acquisition greatly.

In some embodiments, clusters may also be formed by disposing protein nanopores in lipid bilayers supported by solid phase membrane containing an array of apertures. For example, such an array may comprise apertures fabricated (e.g. drilled, etched, or the like) in solid phase support. The geometry of such apertures may vary depending on the fabrication techniques employed. In some embodiments, each such aperture is associated with, or encompassed by, a separate resolution limited area; however, in other embodiments, multiple apertures may be within the same resolution limited area. The cross-sectional area of the apertures may vary widely and may or may not be the same as between different clusters, although such areas are usually substantially the same as a result of conventional fabrication approaches. In some embodiments, apertures have a minimal linear dimension (e.g. diameter in the case of circular apertures) in the range of from 10 to 200 nm, or have areas in the range of from about 100 to 3×10⁴ nm². Across the apertures may be disposed a lipid bilayer. The distribution of protein nanopores per aperture may be varied, for example, by controlling the concentration of protein nanopores during inserting step. In such embodiments, clusters of nanopores may comprise a random number of nanopores. In some embodiments, in which protein nanopores insert randomly into apertures, clusters containing one or more apertures on average have a number of protein nanopores that is greater than zero; in other embodiments, such clusters have a number of protein nanopores that is greater than 0.25; in other embodiments, such clusters have a number of protein nanopores that is greater than 0.5; in other embodiments, such clusters have a number of protein nanopores that is greater than 0.75; in other embodiments, such clusters have a number of protein nanopores that is greater than 1.0.

In some embodiments, methods of the invention use a solid phase membrane, such as a SiN membrane, having an array of apertures therethrough providing communication between a first chamber and a second chamber (also sometimes referred to as a "cis chamber" and a "trans chamber") and supporting a lipid bilayer on a surface facing the second, or trans, chamber. In some embodiments, diameters of the aperture in such a solid phase membrane may be in the range of 10 to 200 nm, or in the range of 20 to 100 nm. In some embodiments, such solid phase membranes further include protein nanopores inserted into the lipid bilayer in regions where such bilayer spans the apertures on the surface facing the trans chamber. In some embodiments, such protein nanopores are inserted from the cis side of the solid phase membrane using techniques described herein. In some embodiments, such protein nanopores have a structure identical to, or similar to, α-hemolysin in that it comprises a barrel, or bore, along an axis and at one end has a "cap" structure and at the other end has a "stem" structure (using the terminology from Song et al, Science, 274: 1859-1866 (1996)). In some embodiments using such protein nanopores, insertion into the lipid bilayer results in the protein nanopore being oriented so that its cap structure is exposed to the cis chamber and its stem structure is exposed to the trans chamber.

In some embodiments, the present invention may employ hybrid nanopores in clusters, particularly for optical-based nanopore sequencing of polynucleotides. Such nanopores comprise a solid-state orifice, or aperture, into which a protein biosensor, such as a protein nanopore, is stably inserted. A charged polymer may be attached to a protein nanopore (e.g. alpha hemolysin) by conventional protein engineering techniques after which an applied electric field may be used to guide a protein nanopore into an aperture in a solid-state membrane. In some embodiments, the aperture in the solid-state substrate is selected to be slightly smaller than the protein, thereby preventing it from translocating through the aperture. Instead, the protein will be embedded into the solid-state orifice.

In some embodiments, a donor fluorophore is attached to the protein nanopore. This complex is then inserted into a solid-state aperture or nanohole (for example, 3-10 nm in diameter) by applying an electric field across the solid state nanohole, or aperture, until the protein nanopore is transported into the solid-state nanohole to form a hybrid nanopore. The formation of the hybrid nanopore can be verified by (a) the inserted protein nanopore causing a drop in current based on a partial blockage of the solid-state nanohole and by (b) the optical detection of the donor fluorophore.

Solid state, or synthetic, nanopores may be prepared in a variety of ways, as exemplified in the references cited above. In some embodiments, a helium ion microscope may be used to drill the synthetic nanopores in a variety of materials, e.g. as disclosed by Yang et al, Nanotechnology, 22: 285310 (2011). A chip that supports one or more regions of a thin-film material, e.g. silicon nitride, that has been processed to be a free-standing membrane is introduced to the helium ion microscope (HIM) chamber. HIM motor controls are used to bring a free-standing membrane into the path of the ion beam while the microscope is set for low magnification. Beam parameters including focus and stigmation are adjusted at a region adjacent to the free-standing membrane, but on the solid substrate. Once the parameters have been properly fixed, the chip position is moved such that the free-standing membrane region is centered on the ion beam scan region and the beam is blanked. The HIM field of view is set to a dimension (in µm) that is sufficient to contain the entire anticipated nanopore pattern and sufficient to be useful in future optical readout (i.e. dependent on optical magnification, camera resolution, etc.). The ion beam is then rastered once through the entire field of view at a pixel dwell time that results in a total ion dose sufficient to remove all or most of the membrane autofluorescence. The field of view is then set to the proper value (smaller than that used above) to perform lithographically-defined milling of either a single nanopore or an array of nanopores. The pixel dwell time of the pattern is set to result in nanopores of one or more predetermined diameters, determined through the use of a calibration sample prior to sample processing. This entire process is repeated for each desired region on a single chip and/or for each chip introduced into the HIM chamber.

In some embodiments, a nanopore may have one or more labels attached for use in optically-based nanopore sequencing methods. The label may be a member of a Forster Resonance Energy Transfer (FRET) pair. Such labels may comprise organic fluorophores, chemiluminescent labels, quantum dots, metallic nanoparticles and/or fluorescent proteins. Target nucleic acids may have one distinct label per nucleotide. The labels attached to the nucleotides may be selected from the group consisting of organic fluorophores. The label attachment site in the pore protein can be generated by conventional protein engineering methods, e.g. a mutant protein can be constructed that will allow the specific binding of the label. As an example, a cysteine residue may be inserted at the desired position of the protein which inserts a thiol (SH) group that can be used to attach a label. The cysteine can either replace a natural occurring amino acid or can be incorporated as an addition amino acid. A maleimide-activated label is then covalently attached to the thiol residue of the protein nanopore. In a preferred embodiment, the attachment of the label to the protein nanopore or the label on the nucleic acid is reversible. By implementing a cleavable crosslinker, an easily breakable chemical bond (e.g. an S-S bond or a pH labile bond) is introduced and the label may be removed when the corresponding conditions are met.

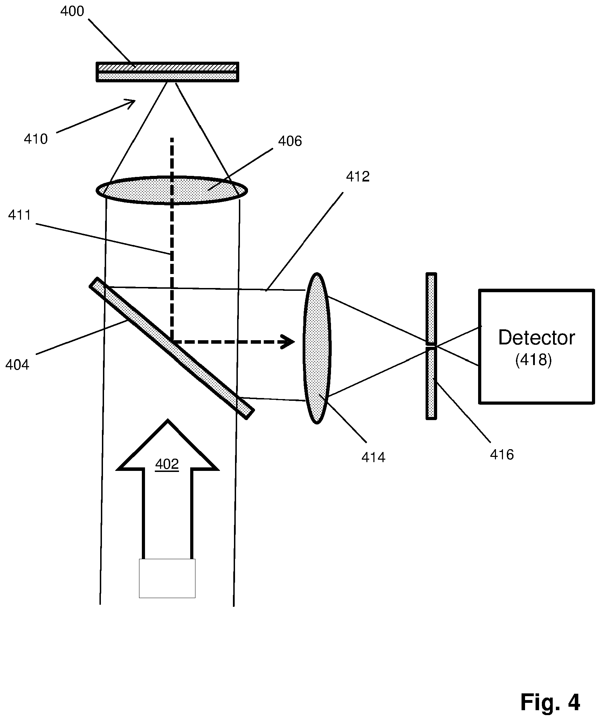

In some embodiments, an epi-illumination system, in which excitation beam delivery and optical signal collection occurs through a single objective, may be used for direct illumination of labels on a polymer analyte or donors on nanopores. The basic components of a confocal epi-illumination system for use with the invention is illustrated in Fig. 4. Excitation beam (402) passes through dichroic (404) and onto objective lens (406) which focuses (410) excitation beam (402) onto layered membrane (400), in which labels are excited directly to emit an optical signal, such as a fluorescent signal, of are excited indirectly via a FRET interaction to emit an optical signal. Such optical signal is collected by objective lens (406) and directed to dichroic (404), which is selected so that it passes light of excitation beam (402) but reflects light of optical signal (411). Reflected optical signals (411) passes through lens (414) which focuses it through pinhole (416) and onto detector (418).

In some embodiments, a device for implementing the above methods for analyzing double stranded polynucleotides typically includes a set of electrodes for establishing an electric field across the layered membrane and nanopores. Single stranded nucleic acids are exposed to nanopores by placing them in an electrolyte in a first chamber, which is configured as the "cis" side of the layered membrane by placement of a negative electrode in the chamber. Upon application of an electric field, the negatively charged single stranded nucleic acids are captured by nanopores and translocated to a second chamber on the other side of the layered membrane, which is configured as the "trans" side of membrane by placement of a positive electrode in the chamber. The speed of translocation depends in part on the ionic strength of the electrolytes in the first and second chambers and the applied voltage across the nanopores. In optically based detection, a translocation speed may be selected by preliminary calibration measurements, for example, using predetermined standards of labeled single stranded nucleic acids that generate signals at different expected rates per nanopore for different voltages. Thus, for DNA sequencing applications, a translocation speed may be selected based on the signal rates from such calibration measurements. Consequently, from such measurements a voltage may be selected that permits, or maximizes, reliable nucleotide identifications, for example, over an array of nanopores. In some embodiments, such calibrations may be made using nucleic acids from the sample of templates being analyzed (instead of, or in addition to, predetermined standard sequences). In some embodiments, such calibrations may be carried out in real time during a sequencing run and the applied voltage may be modified in real time based on such measurements, for example, to maximize the acquisition of nucleotide-specific signals.

Controlling translocation speeds of polymers through nanopores is necessary to permit collection of data from which sequence information can be obtained. Translocation speeds depend in part on the voltage difference (or electrical field strength) across a nanopore and conditions in the reaction mixture of the first chamber where nucleic acid polymers are exposed to the nanopores (e.g. disposed in a solid phase membrane making up one wall of the first chamber). Nucleic acid polymer capture rates by nanopores depend on concentration of such polymers. In some embodiments, conventional reaction mixture conditions for nanopore sequencing may be employed with the invention, for example, 1M KCl (or equivalent salt, such as NaCl, LiCl, or the like) and a pH buffering system (which, for example, ensures that proteins being used, e.g. protein nanopores, nucleases, or the like, are not denatured). In some embodiments, a pH buffering system may be used to keep the pH substantially constant at a value in the range of 6.8 to 8.8. In some embodiments, a voltage difference across the nanopores may be in the range of from 70 to 200 mV. In other embodiments, a voltage difference across the nanopores may be in the range of from 80 to 150 mV. An appropriate voltage for operation may be selected using conventional measurement techniques. Current (or voltage) across a nanopore may readily be measured using commercially available instruments. A voltage difference may be selected so that translocation speed is within a desired range. In some embodiments, a range of translocation speeds comprises those speeds less than 1000 nucleotides per second. In other embodiments, a range of translocation speeds is from 10 to 800 nucleotides per second; in other embodiments, a range of translocation speeds is from 10 to 600 nucleotides per second; in other embodiments, a range of translocation speeds is from 200 to 800 nucleotides per second; in other embodiments, a range of translocation speeds is from 200 to 500 nucleotides per second.

### Labels for Nanopores and Polymers

In some embodiments, a nanopore may be labeled with one or more quantum dots. In particular, in some embodiments, one or more quantum dots may be attached to a nanopore, or attached to a solid phase support adjacent to (and within a FRET distance of an entrance or exit of a nanopore), and employed as donors in FRET reactions with acceptors on analytes. Such uses of quantum dots are well known and are described widely in the scientific and patent literature, such as, in U.S. patents 6,252,303; 6,855,551; 7,235,361.

One example of a Quantum dot which may be utilized as a pore label is a CdTe quantum dot which can be synthesized in an aqueous solution. A CdTe quantum dot may be functionalized with a nucleophilic group such as primary amines, thiols or functional groups such as carboxylic acids. A CdTe quantum dot may include a mercaptopropionic acid capping ligand, which has a carboxylic acid functional group that may be utilized to covalently link a quantum dot to a primary amine on the exterior of a protein pore. The cross-linking reaction may be accomplished using standard cross-linking reagents (homo-bifunctional as well as hetero-bifunctional) which are known to those having ordinary skill in the art of bioconjugation. Care may be taken to ensure that the modifications do not impair or substantially impair the translocation of a nucleic acid through the nanopore. This may be achieved by varying the length of the employed crosslinker molecule used to attach the donor label to the nanopore.

For example, the primary amine of the lysine residue 131 of the natural alpha hemolysin protein (Song, L. et al., Science 274, (1996): 1859-1866) may be used to covalently bind carboxy modified CdTe Quantum dots via 1-Ethyl-3-[3-dimethylaminopropyl]carbodiimide hydrochloride/ N-hydroxysulfosuccinimide (EDC/NHS) coupling chemistry. Alternatively, amino acid 129 (threonine) may be exchanged into cysteine. Since there is no other cysteine residue in the natural alpha hemolysin protein the thiol side group of the newly inserted cysteine may be used to covalently attach other chemical moieties.

A biological polymer, e.g., a nucleic acid molecule or polymer, may be labeled with one or more acceptor labels. For a nucleic acid molecule, each of the four nucleotides or building blocks of a nucleic acid molecule may be labeled with an acceptor label thereby creating a labeled (e.g., fluorescent) counterpart to each naturally occurring nucleotide. The acceptor label may be in the form of an energy accepting molecule which can be attached to one or more nucleotides on a portion or on the entire strand of a converted nucleic acid.

A variety of methods may be utilized to label the monomers or nucleotides of a nucleic acid molecule or polymer. A labeled nucleotide may be incorporated into a nucleic acid during synthesis of a new nucleic acid using the original sample as a template ("labeling by synthesis"). For example, the labeling of nucleic acid may be achieved via PCR, whole genome amplification, rolling circle amplification, primer extension or the like or via various combinations and extensions of the above methods known to persons having ordinary skill in the art.

A label may comprise a reactive group such as a nucleophile (amines, thiols etc.). Such nucleophiles, which are not present in natural nucleic acids, can then be used to attach fluorescent labels via amine or thiol reactive chemistry such as NHS esters, maleimides, epoxy rings, isocyanates etc. Such nucleophile reactive fluorescent dyes (i.e. NHS-dyes) are readily commercially available from different sources. An advantage of labeling a nucleic acid with small nucleophiles lies in the high efficiency of incorporation of such labeled nucleotides when a "labeling by synthesis" approach is used. Bulky fluorescently labeled nucleic acid building blocks may be poorly incorporated by polymerases due to steric hindrance of the labels during the polymerization process into newly synthesized DNA.

Whenever two or more mutually quenching dyes are used, such dyes may be attached to DNA using orthogonal attachment chemistries. For example, NHS esters can be used to react very specifically with primary amines or maleimides will react with thiol groups. Either primary amines (NH₂) or thiol (SH) modified nucleotides are commercially available. These relatively small modifications are readily incorporated in a polymerase mediated DNA synthesis and can be used for subsequent labeling reactions using either NHS or maleimide modified dyes. Guidance for selecting and using such orthogonal linker chemistries may be found in Hermanson (cited above).

Additional orthogonal attachment chemistries for typical attachment positions include Huisgen-type cycloaddition for a copper-catalyzed reaction and an uncatalyzed reaction; alkene plus nitrile oxide cycloaddition, e.g. as disclosed in Gutsmiedl et al, Org. Lett., 11: 2405-2408 (2009); Diels-Alder cycloaddition, e.g. disclosed in Seelig et al, Tetrahedron Lett., 38: 7729-7732 (1997); carbonyl ligation, e.g. as disclosed in Casi et al, J. Am. Chem. Soc., 134: 5887-5892 (2012); Shao et al J. Am. Chem. Soc., 117: 3893-3899 (1995); Rideout, Science, 233: 561-563 (1986); Michael addition, e.g. disclosed in Brinkley, Bioconjugate Chemistry, 3: 2-13 (1992); native chemical ligation, e.g. disclosed in Schuler et al, Bioconjugate Chemistry, 13: 1039-1043 (2002); Dawson et al, Science, 266: 776-779 (1994); or amide formation via an active ester, e.g. disclosed in Hermanson (cited above).

### Definitions

"Evanescent field" means a non-propagating electromagnetic field; that is, it is an electromagnetic field in which the average value of the Poynting vector is zero.

"FRET" or "Förster, or fluorescence, resonant energy transfer" means a non-radiative dipole-dipole energy transfer mechanism from an excited donor fluorophore to an acceptor fluorophore in a ground state. The rate of energy transfer in a FRET interaction depends on the extent of spectral overlap of the emission spectrum of the donor with the absorption spectrum of the acceptor, the quantum yield of the donor, the relative orientation of the donor and acceptor transition dipoles, and the distance between the donor and acceptor molecules, Lakowitz, Principles of Fluorescence Spectroscopy, Third Edition (Springer, 2006). FRET interactions of particular interest are those which result a portion of the energy being transferred to an acceptor, in turn, being emitted by the acceptor as a photon, with a frequency lower than that of the light exciting its donor (i.e. a "FRET signal"). "FRET distance" means a distance between a FRET donor and a FRET acceptor over which a FRET interaction can take place and a detectable FRET signal produced by the FRET acceptor.

"Kit" refers to any delivery system for delivering materials or reagents for carrying out a method of the invention. In the context of reaction assays, such delivery systems include systems that allow for the storage, transport, or delivery of reaction reagents (e.g., fluorescent labels, such as mutually quenching fluorescent labels, fluorescent label linking agents, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. Such contents may be delivered to the intended recipient together or separately. For example, a first container may contain an enzyme for use in an assay, while a second or more containers contain mutually quenching fluorescent labels.

"Nanopore" means any opening positioned in a substrate that allows the passage of analytes through the substrate in a predetermined or discernable order, or in the case of polymer analytes, passage of their monomeric units through the substrate in a predetermined or discernible order. In the latter case, a predetermined or discernible order may be the primary sequence of monomeric units in the polymer. Examples of nanopores include proteinaceous or protein based nanopores, synthetic or solid state nanopores, and hybrid nanopores comprising a solid state nanopore having a protein nanopore embedded therein. A nanopore may have an inner diameter of 1-10 nm or 1-5 nm or 1-3 nm. Examples of protein nanopores include but are not limited to, alpha-hemolysin, voltage-dependent mitochondrial porin (VDAC), OmpF, OmpC, MspA and LamB (maltoporin), e.g. disclosed in Rhee, M. et al., Trends in Biotechnology, 25(4) (2007): 174-181; Bayley et al (cited above); Gundlach et al, U.S. patent publication 2012/0055792; and the like. Any protein pore that allows the translocation of single nucleic acid molecules may be employed. A nanopore protein may be labeled at a specific site on the exterior of the pore, or at a specific site on the exterior of one or more monomer units making up the pore forming protein. Pore proteins are chosen from a group of proteins such as, but not limited to, alpha-hemolysin, MspA, voltage-dependent mitochondrial porin (VDAC), Anthrax porin, OmpF, OmpC and LamB (maltoporin). Integration of the pore protein into the solid state hole is accomplished by attaching a charged polymer to the pore protein. After applying an electric field the charged complex is electrophoretically pulled into the solid state hole. A synthetic nanopore, or solid-state nanopore, may be created in various forms of solid substrates, examples of which include but are not limited to silicones (e.g. Si3N4, SiO2), metals, metal oxides (e.g. Al2O3) plastics, glass, semiconductor material, and combinations thereof. A synthetic nanopore may be more stable than a biological protein pore positioned in a lipid bilayer membrane. A synthetic nanopore may also be created by using a carbon nanotube embedded in a suitable substrate such as but not limited to polymerized epoxy. Carbon nanotubes can have uniform and well-defined chemical and structural properties. Various sized carbon nanotubes can be obtained, ranging from one to hundreds of nanometers. The surface charge of a carbon nanotube is known to be about zero, and as a result, electrophoretic transport of a nucleic acid through the nanopore becomes simple and predictable (Ito, T. et al., Chem. Commun. 12 (2003): 1482-83). The substrate surface of a synthetic nanopore may be chemically modified to allow for covalent attachment of the protein pore or to render the surface properties suitable for optical nanopore sequencing. Such surface modifications can be covalent or non-covalent. Most covalent modification include an organosilane deposition for which the most common protocols are described: 1) Deposition from aqueous alcohol. This is the most facile method for preparing silylated surfaces. A 95% ethanol-5% water solution is adjusted to pH 4.5-5.5 with acetic acid. Silane is added with stirring to yield a 2% final concentration. After hydrolysis and silanol group formation the substrate is added for 2-5min. After rinsed free of excess materials by dipping briefly in ethanol. Cure of the silane layer is for 5-10min at 110 degrees Celsius. 2) Vapor Phase Deposition. Silanes can be applied to substrates under dry aprotic conditions by chemical vapor deposition methods. These methods favor monolayer deposition. In closed chamber designs, substrates are heated to sufficient temperature to achieve 5mm vapor pressure. Alternatively, vacuum can be applied until silane evaporation is observed. 3) Spin-on deposition. Spin-on applications can be made under hydrolytic conditions which favor maximum functionalization and polylayer deposition or dry conditions which favor monolayer deposition. In some embodiments, single nanopores are employed with methods of the invention. In other embodiments, a plurality of nanopores are employed. In some of the latter embodiments, a plurality of nanopores is employed as an array of nanopores, usually disposed in a planar substrate, such as a solid phase membrane. Nanopores of a nanopore array may be spaced regularly, for example, in a rectilinear pattern, or may be spaced randomly. In a preferred embodiment, nanopores are spaced regularly in a rectilinear pattern in a planar solid phase substrate.

"Nanostructure" (used interchangeably with "nanoscale structure" and "nanoscale feature") means a structure that has at least one dimension within a range of a few nanometers to several hundred nanometers, for example, from 1 to 1000 nanometers. In some applications, such range is from 2 to 500 nanometers; in other applications, such range is from 3 to 500 nanometers. The shape and geometry of nanostructures may vary widely and include, but are not limited to, nanopores, nanowells, nanoparticles, and any other convenient shapes particularly suitable for carrying out sequences of reactions. In some embodiments, nanostructures may be protein nanopores operationally associated with a solid phase membrane. Some nanostructures, such as, nanopores and nanowells, may be formed in a larger common substrate, such as a solid phase membrane, or other solid, to form arrays of nanopores or nanowells. Nanostructures of particular interest are those capable of supporting or containing a chemical, physical (e.g. FRET), enzymatic and/or binding reaction or a sequence of such reactions. In some embodiments, a nanostructure, such as a nanowell, encloses a volume that is less than one nanoliter (10×-9 liter), less than one picoliter, or less than one femtoliter. In other embodiments, each of the individual nanowells provides a volume that is less than 1000 zeptoliters, 100 zeptoliters, 80 zeptoliters, or less than 50 zeptoliters, or less than 1 zeptoliter, or even less than 100 yactoliters. In some embodiments, nanowells comprise zero mode waveguides.

"Polymer" means a plurality of monomers connected into a linear chain. Usually, polymers comprise more than one type of monomer, for example, as a polynucleotide comprising A's, C's, G's and T's, or a polypeptide comprising more than one kind of amino acid. Monomers may include without limitation nucleosides and derivatives or analogs thereof and amino acids and derivatives and analogs thereof. In some embodiments, polymers are polynucleotides, whereby nucleoside monomers are connected by phosphodiester linkages, or analogs thereof.

"Polynucleotide" or "oligonucleotide" are used interchangeably and each mean a linear polymer of nucleotide monomers. Monomers making up polynucleotides and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, or the like. Such monomers and their internucleosidic linkages may be naturally occurring or may be analogs thereof, e.g. naturally occurring or non-naturally occurring analogs. Non-naturally occurring analogs may include PNAs, phosphorothioate internucleosidic linkages, bases containing linking groups permitting the attachment of labels, such as fluorophores, or haptens, and the like. Whenever the use of an oligonucleotide or polynucleotide requires enzymatic processing, such as extension by a polymerase, ligation by a ligase, or the like, one of ordinary skill would understand that oligonucleotides or polynucleotides in those instances would not contain certain analogs of internucleosidic linkages, sugar moieties, or bases at any or some positions. Polynucleotides typically range in size from a few monomeric units, e.g. 5-40, when they are usually referred to as "oligonucleotides," to several thousand monomeric units. Whenever a polynucleotide or oligonucleotide is represented by a sequence of letters (upper or lower case), such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, "I" denotes deoxyinosine, "U" denotes uridine, unless otherwise indicated or obvious from context. Unless otherwise noted the terminology and atom numbering conventions will follow those disclosed in Strachan and Read, Human Molecular Genetics 2 (Wiley-Liss, New York, 1999). Usually polynucleotides comprise the four natural nucleosides (e.g. deoxyadenosine, deoxycytidine, deoxyguanosine, deoxythymidine for DNA or their ribose counterparts for RNA) linked by phosphodiester linkages; however, they may also comprise non-natural nucleotide analogs, e.g. including modified bases, sugars, or internucleosidic linkages. It is clear to those skilled in the art that where an enzyme has specific oligonucleotide or polynucleotide substrate requirements for activity, e.g. single stranded DNA, RNA/DNA duplex, or the like, then selection of appropriate composition for the oligonucleotide or polynucleotide substrates is well within the knowledge of one of ordinary skill, especially with guidance from treatises, such as Sambrook et al, Molecular Cloning, Second Edition (Cold Spring Harbor Laboratory, New York, 1989), and like references. Likewise, the oligonucleotide and polynucleotide may refer to either a single stranded form or a double stranded form (i.e. duplexes of an oligonucleotide or polynucleotide and its respective complement). It will be clear to one of ordinary skill which form or whether both forms are intended from the context of the terms usage.

"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references: Dieffenbach, editor, PCR Primer: A Laboratory Manual, 2nd Edition (Cold Spring Harbor Press, New York, 2003).

"Resolution limited area" is an area of a surface of a nanopore or nanowell array within which individual features or light emission sources cannot be distinguished by an optical signal detection system. Without intending to be limited by theory, such resolution limited area is determined by a resolution limit (also sometimes referred to as a "diffraction limit" or "diffraction barrier") of an optical system. Such limit is determined by the wavelength of the emission source and the optical components and may be defined by d=λ/NA, where d is the smallest feature that can be resolved, λ is the wavelength of the light and NA is the numerical aperture of the objective lens used to focus the light. Thus, whenever two or more nanopores are within a resolution limited area and two or more optical signals are generated at the respective nanopores, an optical detection system cannot distinguish or determine which optical signals came from which nanopore. In accordance with the invention, a surface of a nanopore array may be partitioned, or subdivided, into non-overlapping regions, or substantially non-overlapping regions, corresponding to resolution limited areas. The size of such subdivisions corresponding to resolution limited areas may depend on a particular optical detection system employed. In some embodiments, whenever light emission sources are within the visible spectrum, a resolution limited area is in the range of from 300 nm² to 3.0 µm²; in other embodiments, a resolution limited area is in the range of from 1200 nm² to 0.7 µm²; in other embodiments, a resolution limited area is in the range of from 3×10⁴ nm² to 0.7 µm², wherein the foregoing ranges of areas are in reference to a surface of a nanopore or nanowell array. In some embodiments, the visible spectrum means wavelengths in the range of from about 380 nm to about 700 nm.

"Sequence determination", "sequencing" or "determining a nucleotide sequence" or like terms in reference to polynucleotides includes determination of partial as well as full sequence information of the polynucleotide. That is, the terms include sequences of subsets of the full set of four natural nucleotides, A, C, G and T, such as, for example, a sequence of just A's and C's of a target polynucleotide. That is, the terms include the determination of the identities, ordering, and locations of one, two, three or all of the four types of nucleotides within a target polynucleotide. In some embodiments, the terms include the determination of the identities, ordering, and locations of two, three or all of the four types of nucleotides within a target polynucleotide. In some embodiments sequence determination may be accomplished by identifying the ordering and locations of a single type of nucleotide, e.g. cytosines, within the target polynucleotide "catcgc ..." so that its sequence is represented as a binary code, e.g. "100101 ... " representing "c-(not c)(not c)c-(not c)-c . . . " and the like. In some embodiments, the terms may also include subsequences of a target polynucleotide that serve as a fingerprint for the target polynucleotide; that is, subsequences that uniquely identify a target polynucleotide, or a class of target polynucleotides, within a set of polynucleotides, e.g. all different RNA sequences expressed by a cell.

## Claims

1. A method of analyzing double stranded polynucleotides comprising:
copying a strand of a double stranded polynucleotide so that nucleotide analogs with distinct optical labels are substituted for two or three kinds of nucleotide to form a labeled strand;
copying a complement of the strand so that said nucleotide analogs are substituted for the same two or three kinds of nucleotide to form a labeled complement;
translocating the labeled strand through a nanopore so that the nucleotides of the labeled strand pass single file through an excitation zone where optical labels are excited to generate optical signals;
detecting a time series of optical signals from the optical labels as the labeled strand translocates through the nanopore to produce a strand optical signature;
translocating the labeled complement through a nanopore so that the nucleotides of the labeled complement pass single file through an excitation zone where optical labels are excited to generate optical signals;
detecting a time series of optical signals from the optical labels as the labeled complement translocates through the nanopore to produce a complement optical signature;
determining a sequence of the double stranded polynucleotide from the strand optical signature and the complement optical signature.

2. The method of claim 1, wherein said step of determining includes pairing said strand optical signature with said complement optical signature by complementarity of said optical signatures.

3. The method of claim 1, wherein either (i) at least one of said two or three kinds of nucleotide is a pyrimidine, (ii) two of said two or three kinds of nucleotide are pyrimidines, or (iii) two of said two or three kinds of nucleotide are purines.

4. The method of claim 1, wherein either:
a) said steps of copying are carried out by a primer extension reaction, optionally wherein the method further includes a step of ligating a hairpin adaptor to an end of said double stranded polynucleotide so that said strand and said complement may be copied by a single primer extension; or
b) said steps of copying includes substituting said nucleotide analogs for every one of said two or three kinds of nucleotide to form said labeled strand and said labeled complement.

5. The method of claim 1, wherein the method is a method of analyzing double stranded polynucleotides of a population, the method comprising the steps of:
copying a strand of each double stranded polynucleotide so that nucleotide analogs with distinct optical labels are substituted for two or three kinds of nucleotide to form a labeled strand, each double stranded polynucleotide having a predetermined minimal length;
copying a complement of the strand so that said nucleotide analogs are substituted for the same two or three kinds of nucleotide to form a labeled complement;
separately translocating the labeled strand and the labeled complement through a nanopore so that the nucleotides of the labeled strand and the labeled complement pass single file through an excitation zone where optical labels are excited to generate optical signals;
separately detecting a time series of optical signals from the optical labels as the labeled strand translocates through the nanopore to produce a strand optical signature and a time series of optical signals from the optical labels as the labeled complement translocates through the nanopore to produce a complement optical signature;
associating the strand optical signature and the complement optical signature from each double stranded polynucleotide, wherein the predetermined minimal length is selected so that such association is correct with a probability of at least ninety percent.
determining a sequence of each double stranded polynucleotide from the associated strand optical signature and the complement optical signature.

6. The method of claim 5, wherein said population comprises DNA fragments of a human genome, a bacterial genome, or a genome of a single-celled eukaryotic organism.

7. The method of claim 6, wherein said DNA fragments are each at least 100 basepairs in length, optionally wherein said DNA fragments are in the range of from 100 to 10,000 basepairs in length.

8. The method of claim 5, wherein said population comprises less than 10¹⁵ distinct double stranded DNA.

9. The method of any one of claims 1 to 8, wherein the two kinds of nucleotide are C and T, C and G, C and A, T and G, T and A, or G and A.

10. The method of any one of claims 1 to 9, wherein a full sequence of the polynucleotide is determined.

## Patentansprüche

1. Verfahren zur Analyse doppelsträngiger Polynukleotide mit den Schritten:
Kopieren eines doppelsträngigen Polynukleotidstrangs, so dass Nukleotidanaloga mit unterschiedlichen optischen Markierungen zwei oder drei Arten von Nukleotiden ersetzen, um einen markierten Strang zu bilden;
Kopieren eines Komplements des Strangs, so dass die Nukleotidanaloga durch die gleichen zwei oder drei Arten von Nukleotiden ersetzt werden, um ein markiertes Komplement zu bilden;
Translozieren des markierten Strangs durch eine Nanopore, so dass die Nukleotide des markierten Strangs einzeln durch eine Anregungszone transloziert werden, in der optische Markierungen angeregt werden, um optische Signale zu erzeugen;
Erfassen einer Zeitreihe optischer Signale von den optischen Markierungen, während der markierte Strang durch die Nanopore transloziert wird, um eine optische Signatur des Strangs zu erzeugen;
Translozieren des markierten Komplements durch eine Nanopore, so dass die Nukleotide des markierten Komplements einzeln durch eine Anregungszone wandern, in der optische Markierungen angeregt werden, um optische Signale zu erzeugen;
Erfassen einer Zeitreihe optischer Signale von den optischen Markierungen, während das markierte Komplement durch die Nanopore transloziert wird, um eine optische Signatur des Komplements zu erzeugen;
Bestimmen einer Sequenz des doppelsträngigen Polynukleotids aus der optischen Signatur des Strangs und der optischen Signatur des Komplements.

2. Verfahren nach Anspruch 1, bei dem der Schritt des Bestimmens das Paaren der optischen Signatur des Strangs mit der optischen Signatur des Komplements durch Komplementarität der optischen Signaturen umfasst.

3. Verfahren nach Anspruch 1, bei dem entweder (i) mindestens eine der zwei oder drei Arten von Nukleotiden ein Pyrimidin ist, (ii) zwei der zwei oder drei Arten von Nukleotiden Pyrimidine sind oder (iii) zwei der zwei oder drei Arten von Nukleotiden Purine sind.

4. Verfahren nach Anspruch 1, bei dem entweder
a) die Schritte des Kopierens durch eine Primerverlängerungsreaktion durchgeführt werden, wobei optional
das Verfahren ferner einen Schritt des Ligierens eines Haarnadel-Adapters an ein Ende des doppelsträngigen Polynukleotids umfasst, so dass der Strang und das Komplement durch eine einzige Primerverlängerung kopiert werden können; oder
b) die Schritte des Kopierens das Ersetzen der zwei oder drei Arten von Nukleotiden durch die Nukleotidanaloga umfassen, um den markierten Strang und das markierte Komplement zu bilden.

5. Verfahren nach Anspruch 1, wobei das Verfahren ein Verfahren zur Analyse doppelsträngiger Polynukleotide einer Population ist, und das Verfahren die folgenden Schritte umfasst:
Kopieren eines Strangs jedes doppelsträngigen Polynukleotids, so dass Nukleotidanaloga mit unterschiedlichen optischen Markierungen anstelle von zwei oder drei Arten von Nukleotiden eingesetzt werden, um einen markierten Strang zu bilden, wobei jedes doppelsträngige Polynukleotid eine vorgegebene Mindestlänge aufweist;
Kopieren eines Komplements des Strangs, so dass die Nukleotidanaloga durch die gleichen zwei oder drei Arten von Nukleotiden ersetzt werden, um ein markiertes Komplement zu bilden;
getrenntes Translozieren des markierten Strangs und des markierten Komplements durch eine Nanopore, so dass die Nukleotide des markierten Strangs und des markierten Komplements einzeln durch eine Anregungszone wandern, in der optische Markierungen angeregt werden, um optische Signale zu erzeugen;
getrenntes Erfassen einer Zeitreihe optischer Signale von den optischen Markierungen, während der markierte Strang durch die Nanopore transloziert wird, um eine optische Signatur des Strangs zu erzeugen, und einer Zeitreihe optischer Signale von den optischen Markierungen, während das markierte Komplement durch die Nanopore transloziert wird, um eine optische Signatur des Komplements zu erzeugen;
Verknüpfen der optischen Signatur des Strangs und der optischen Signatur des Komplements von jedem doppelsträngigen Polynukleotid, wobei die vorgegebene Mindestlänge so gewählt wird, dass eine solche Verknüpfung mit einer Wahrscheinlichkeit von mindestens 90 % korrekt ist;
Bestimmen einer Sequenz jedes doppelsträngigen Polynukleotids aus der zugehörigen optischen Signatur des Strangs und der optischen Signatur des Komplements.

6. Verfahren nach Anspruch 5, bei dem die Population DNA-Fragmente eines menschlichen Genoms, eines bakteriellen Genoms oder eines Genoms eines einzelligen eukaryotischen Organismus umfasst.

7. Verfahren nach Anspruch 6, bei dem die DNA-Fragmente jeweils eine Länge von mindestens 100 Basenpaare haben, wobei optional die Längen der DNA-Fragmente im Bereich von 100 bis 10.000 Basenpaaren liegen.

8. Verfahren nach Anspruch 5, bei dem die Population weniger als 10¹⁵ verschiedene doppelsträngige DNA umfasst.

9. Verfahren nach einem der Ansprüche 1 bis 8, bei dem die beiden Arten von Nukleotiden C und T, C und G, C und A, T und G, T und A oder G und A sind.

10. Verfahren nach einem der Ansprüche 1 bis 9, bei dem eine vollständige Sequenz des Polynukleotids bestimmt wird.

## Revendications

1. Procédé d'analyse de polynucléotides double brin comprenant :
la copie d'un brin d'un polynucléotide double brin de telle sorte que des analogues nucléotidiques avec des marqueurs optiques distincts sont substitués à deux ou trois types de nucléotide pour former un brin marqué ;
la copie d'un complément du brin de telle sorte que lesdits analogues nucléotidiques sont substitués aux deux ou trois mêmes types de nucléotide pour former un complément marqué ;
la translocation du brin marqué à travers un nanopore de telle sorte que les nucléotides du brin marqué passent en file indienne à travers une zone d'excitation où des marqueurs optiques sont excités pour générer des signaux optiques ;
la détection d'une série temporelle de signaux optiques provenant des marqueurs optiques lorsque le brin marqué est transloqué à travers le nanopore pour produire une signature optique de brin ;
la translocation du complément marqué à travers un nanopore de telle sorte que les nucléotides du complément marqué passent en file indienne à travers une zone d'excitation où des marqueurs optiques sont excités pour générer des signaux optiques ;
la détection d'une série temporelle de signaux optiques provenant des marqueurs optiques lorsque le complément marqué est transloqué à travers le nanopore pour produire une signature optique de complément ;
la détermination d'une séquence du polynucléotide double brin à partir de la signature optique de brin et de la signature optique de complément.

2. Procédé selon la revendication 1, dans lequel ladite étape de détermination comporte l'appariement de ladite signature optique de brin avec ladite signature optique de complément par complémentarité desdites signatures optiques.

3. Procédé selon la revendication 1, dans lequel (i) au moins un desdits deux ou trois types de nucléotide est une pyrimidine, (ii) deux desdits deux ou trois types de nucléotide sont des pyrimidines, ou (iii) deux desdits deux ou trois types de nucléotide sont des purines.

4. Procédé selon la revendication 1, dans lequel :
a) lesdites étapes de copie sont réalisées par une réaction d'extension d'amorce, dans lequel facultativement le procédé comporte en outre une étape de ligature d'un adaptateur en épingle à cheveux à une extrémité dudit polynucléotide double brin de sorte que ledit brin et ledit complément peuvent être copiés par une extension d'amorce unique ; ou
b) lesdites étapes de copie comportent la substitution desdits analogues nucléotidiques à chacun desdits deux ou trois types de nucléotide pour former ledit brin marqué et ledit complément marqué.

5. Procédé selon la revendication 1, dans lequel le procédé est un procédé d'analyse de polynucléotides double brin d'une population, le procédé comprenant les étapes consistant à :
copier un brin de chaque polynucléotide double brin de telle sorte que des analogues nucléotidiques avec des marqueurs optiques distincts sont substitués à deux ou trois types de nucléotides pour former un brin marqué, chaque polynucléotide double brin ayant une longueur minimale prédéterminée ;
copier un complément du brin de telle sorte que lesdits analogues nucléotidiques sont substitués aux deux ou trois mêmes types de nucléotide pour former un complément marqué ;
transloquer séparément le brin marqué et le complément marqué à travers un nanopore de telle sorte que les nucléotides du brin marqué et du complément marqué passent en file indienne à travers une zone d'excitation où des marqueurs optiques sont excités pour générer des signaux optiques ;
détecter séparément une série temporelle de signaux optiques provenant des marqueurs optiques lorsque le brin marqué est transloqué à travers le nanopore pour produire une signature optique de brin et une série temporelle de signaux optiques provenant des marqueurs optiques lorsque le complément marqué est transloqué à travers le nanopore pour produire un optique signature de complément ;
associer la signature optique de brin et la signature optique de complément de chaque polynucléotide double brin, dans lequel la longueur minimale prédéterminée est sélectionnée de telle sorte qu'une telle association est correcte avec une probabilité d'au moins quatre-vingt-dix pour cent,
déterminer une séquence de chaque polynucléotide double brin à partir de la signature optique de brin associé et de la signature optique de complément.

6. Procédé selon la revendication 5, dans lequel ladite population comprend des fragments d'ADN d'un génome humain, d'un génome bactérien, ou d'un génome d'un organisme eucaryote unicellulaire.

7. Procédé selon la revendication 6, dans lequel lesdits fragments d'ADN ont chacun une longueur d'au moins 100 paires de bases, facultativement dans lequel lesdits fragments d'ADN ont une longueur dans la plage de 100 à 10 000 paires de bases.

8. Procédé selon la revendication 5, dans lequel ladite population comprend moins de 10¹⁵ ADN double brin distincts.

9. Procédé selon l'une quelconque des revendications 1 à 8, dans lequel les deux types de nucléotide sont C et T, C et G, C et A, T et G, T et A, ou G et A.

10. Procédé selon l'une quelconque des revendications 1 à 9, dans lequel une séquence complète du polynucléotide est déterminée.
